# EUROPÄISCHE PATENTSCHRIFT

(11) **EP 4 000 414 B1**
(45) Veröffentlichungstag und Bekanntmachung des Hinweises auf die Patenterteilung: **20.05.2026**
(21) Anmeldenummer: 20209040.3
(22) Anmeldetag: 20.11.2020
(51) Int. Cl.: A23L 29/00, A23L 33/105, C07C 51/00, C07C 51/47, C07C 65/03, C11B 5/00, C12N 9/18, C12P 7/42, A23B 70/10

(54) **VERFAHREN ZUR HERSTELLUNG EINES EXTRAKTS ENTHALTEND GALLUSSÄURE, WÄSSRIGES KONZENTRAT ENTHALTEND GALLUSSÄURE SOWIE VERWENDUNG DES WÄSSRIGEN KONZENTRATS IN LEBENSMITTEL UND NAHRUNGSERGÄNZUNGSMITTELN**
METHOD FOR PRODUCING AN EXTRACT CONTAINING GALLIC ACID, AQUEOUS CONCENTRATE CONTAINING GALLIC ACID AND USE OF THE AQUEOUS CONCENTRATE IN FOOD AND DIETARY SUPPLEMENTS
PROCÉDÉ DE PRÉPARATION D'UN EXTRAIT CONTENANT DE L'ACIDE GALLIQUE, CONCENTRÉ AQUEUX CONTENANT DE L'ACIDE GALLIQUE, ET UTILISATION DU CONCENTRÉ AQUEUX DANS DES ALIMENTS ET DES COMPLÉMENTS ALIMENTAIRES

(43) Veröffentlichungstag der Anmeldung: 25.05.2022
(73) Patentinhaber: Red Bull GmbH, 5330 Fuschl am See (AT)
(72) Erfinder: Nachbagauer, Josef, 5330 Fuschl am See (AT); Jenny, Marcel, 5330 Fuschl am See (AT); Rinderer, Christian, 5330 Fuschl am See (AT); Winter, Isabella, 5330 Fuschl am See (AT)
(74) Vertreter: Metten, Karl-Heinz

(56) Entgegenhaltungen:
- EP-A1- 2 022 346
- EP-A1- 2 606 902
- EP-B1- 2 022 346
- WO-A1-00/15044
- CN-A- 107 312 768
- JP-B2- 4 630 295
- CHAKRABORTY A ET AL: "Evaluation of biological activities of Rhus aromaticae extracts", PHARMACEUTICAL AND PHARMACOLOGICAL LETTERS, MEDPHARM, SCIENTIFIC PUBL., STUTTGART, DE, vol. 10, no. 2, 2000, pages 76 - 81, XP009526924, ISSN: 0939-9488
- ANNETTE ABHAU: "The survival of a Malagasy lemur species Propithecus verreauxi coquereli in captivity: The vital role of a self-selected plant diet", 20 June 2007 (2007-06-20), pages 1 - 325, XP055030205, Retrieved from the Internet <URL:http://d-nb.info/984680519/34> [retrieved on 20120618]
- KAR B ET AL: "MICROBIAL PRODUCTION OF GALLIC ACID BY MODIFIED SOLID STATE FERMENTATION", JOURNAL OF INDUSTRIAL MICROBIOLOGY & BIOTECHNOLOGY, BASINGSTOKE, GB, vol. 23, no. 3, September 1999 (1999-09-01), pages 173 - 177, XP009017209, ISSN: 1367-5435, DOI: 10.1038/SJ.JIM.2900713
- POURRAT H ET AL: "MICROBIOLOGICAL PRODUCTION OF GALLIC ACID FROM RHUS CORIARIA L", BIOTECHNOLOGY LETTERS, vol. 9, no. 10, 1987, Dordrecht, pages 731 - 734, XP055794342, ISSN: 0141-5492
- MARTINS ISABELA M ET AL: "Tannase enhances the anti-inflammatory effect of grape pomace in Caco-2 cells treated with IL-1[beta]", JOURNAL OF FUNCTIONAL FOODS, ELSEVIER BV, NL, vol. 29, 19 December 2016 (2016-12-19), pages 69 - 76, XP029886766, ISSN: 1756-4646, DOI: 10.1016/J.JFF.2016.12.011

## Beschreibung

Die vorliegende Erfindung betrifft ein Verfahren zur Herstellung eines Extrakts enthaltend Gallussäure, insbesondere in hohen Konzentrationen. Ferner betrifft die Erfindung ein wässriges Konzentrat enthaltend Gallussäure. Außerdem betrifft die Erfindung die Verwendung des erfindungsgemäßen Konzentrats in Lebensmitteln und Nahrungsergänzungsmitteln.

Gallussäure (3,4,5-Trihydroxybenzoesäure) ist Säurekomponente der Gallotannine, bei denen es sich um hydrolysierbare Gerbstoffe handelt. Sie kommen z.B. in der Eichenrinde und in Galläpfeln vor. Gallussäure wird für die Herstellung von Antioxidantien, Sonnenschutzmitteln und Farbstoffen eingesetzt. Des Weiteren wurde postuliert, dass die Gallussäure aufgrund ihrer hypoglykämischen Eigenschaften bei adipösen Personen, die gallussäurereiche Nahrungsmittel zu sich nehmen, nachteilige gesundheitliche Auswirkungen verhindern helfen soll. Mit dem Verzehr von Gallussäure sollen ein nachweisbarer DNA-Schutz bzw. eine verringerte Oxidation von DNA-Basen sowie reduzierte Entzündungsparameter einhergehen (s.a. T. Setayesh et al. "Gallic acid, a common dietary phenolic protects against high fat diet induced DNA damage" in European Journal of Nutrition, Vol. 58, Seiten 2315 bis 26, 2019).

Gemäß der EP 1 942 919 B1 soll eine Zusammensetzung, enthaltend einen Polyphenolextrakt aus Trauben, der 400 bis 1500 ppm Gallussäure aufweisen kann, zur Behandlung von Metabolischem Syndrom oder Prähypertonie geeignet sein.

Gemäß der EP 2 095 718 B1 soll sich ein Teegetränk, dass auf einen Tee-Extrakt zurückgeht, in dem das Gewichtsverhältnis von Gallussäure zu nicht-polymeren Catechinen nicht größer als o,3 sein darf, durch eine verringerte Bitterkeit und einen reduzierten sauren Geschmack auszeichnen.

In der EP 2 143 344 B1 wird eine Getränkezusammensetzung beschrieben, enthaltend 0,5 bis 25,0 Gew.-% nicht-polymere Catechine, ein Kohlenhydrat und eine Hydroxycarbonsäure. In diesen Zusammensetzungen hat der Gehalt an Gallussäure niedriger als 0,6 Gew.-% zu sein.

B. Kar et al. (Journal of Industrial Microbiology & Biotechnology (1999) 23, 173-177, "Microbial production of gallic acid by modified solid state fermentation") beschreiben die Biokonversion von Tannin zu Gallussäure aus dem Pulver von Terischoten durch den Pilz *Rhizopus oryzae* in einem Bioreaktor mit einem perforierten Schwimmer für feste Substrate und induziertes Inokulieren. Für die Herstellung des Inokulums wurde ein Czapek-Dox-Medium mit 2 % Tanninsäure als einzige Kohlenstoffquelle verwendet. Gallussäure wurde aus der fermentierten Biomasse durch Extraktion mit Diethylether gewonnen. Die JP 4630295 B2 offenbart ein Verfahren zur Herstellung eines Tee-Extrakts, bei dem in einem ersten Schritt das Tee-Rohmaterial in Wasser in Gegenwart von Tannase extrahiert wird. Anschließend wird Ethanol hinzugegeben, um die Tannase zu deaktivieren. I.M. Martins et al. (Journal of Functional Foods 29 (2017) 69-76, "Tannase enhances the antiinflammatory effect of grape pomace in Caco-2 cells treated with IL-1β") beschreiben den Einsatz von Methanol zur Deaktivierung von Tannase.

Gallussäure wird bislang häufig mithilfe von wässrigen Extraktionsverfahren gewonnen. Diese Verfahren führen jedoch regelmäßig nur zu geringen Ausbeuten an Gallussäure und sind material-, apparate- und kostenintensiv.

Der vorliegenden Erfindung lag daher die Aufgabe zugrunde, ein Verfahren zur Gewinnung von Gallussäure verfügbar zu machen, das nicht mit den Nachteilen des Stands der Technik behaftet ist und das insbesondere Gallussäure verlässlich in guten Ausbeuten auf effiziente Weise verfügbar macht.

Demgemäß wurde ein Verfahren zur Herstellung eines Extrakts enthaltend Gallussäure gefunden (auch erste Ausführungsvariante des erfindungsgemäßen Verfahrens genannt), umfassend die Schritte
a) Zurverfügungstellung von, insbesondere zerkleinerten und/oder, insbesondere und, getrockneten, Pflanzen oder Pflanzenbestandteilen, enthaltend Tannine, insbesondere Tanninsäure,
b) Zurverfügungstellung des Enzyms Tannase (IUB 3.1.1.20),
c) Zurverfügungstellung von Ethanol,
d) gegebenenfalls Zerkleinern bzw. weiteres Zerkleinern der Pflanzen oder Pflanzenbestandteile gemäß Schritt a) und
e) extrahierendes Behandeln der gemäß Schritt a) und/oder d), insbesondere oder, erhaltenen, insbesondere zerkleinerten, Pflanzen oder Pflanzenbestandteile mit Wasser in Gegenwart des Enzyms Tannase (IUB 3.1.1.20) bei Temperaturen im Bereich von 35 bis 40 °C unter Erhalt eines Produktgemisches enthaltend Gallussäure,
f) das Abtrennen der, insbesondere zerkleinerten, Pflanzen oder Pflanzenbestandteile aus dem gemäß Schritt e) erhaltenen Produktgemisches, insbesondere im Anschluss an Schritt e) und
g) das evaporative Konzentrieren des gemäß Schritt f) erhaltenen Produktgemisches, enthaltend Gallussäure im Anschluss an Schritt f) und vor Schritt h) und
h) Behandeln des Produktgemisches gemäß Schritt e) oder f) mit Ethanol und Behandeln des Produktgemisches gemäß Schritt e) oder f) oder g) mit Wärme bei Temperaturen im Bereich von 40 bis 75 °C, wobei Schritt h) bewirkt, das Enzym Tannase zu deaktivieren, wie auch Proteine und/oder Polysaccharide zu denaturieren und auszufällen, und
i) Filtrieren des behandelten Produktgemisches gemäß Schritt h) und,
j) Entfernen des in Schritt h) zugegebenen Ethanols aus dem behandelten Produktgemisch gemäß Schritt h) oder dem filtrierten Produktgemisch gemäß Schritt i),
k) evaporatives Konzentrieren des nach Schritt j) erhaltenen wässrigen Extrakts, enthaltend Gallussäure unter Erhalt eines wässrigen Konzentrats,
   wobei in Schritt h) Ethanol in einer Menge im Bereich von 40 bis 60 Gewichtsprozent, bezogen auf das Gesamtgewicht aus dem Ethanol und dem Wasser bzw. dem wässrigen System, zugegeben wird, wobei die, insbesondere gemahlenen, vorzugsweise zerkleinerten, Pflanzen oder Pflanzenbestandteile in Schritt e) immobilisiert vorliegen und
   wobei das extrahierende Behandeln gemäß Schritt e) ein Perkolationsverfahren umfasst.

Bei den, insbesondere zerkleinerten und/oder getrockneten, Pflanzen oder Pflanzenbestandteilen kann zurückgegriffen werden auf Blätter, Hölzer, Rinden, Früchte, Samen und/oder Wurzeln, vorzugsweise den Früchten, von Hülsenfrüchten, z.B. Bohnen und Erbsen; Nüssen, z.B. Walnüssen (*Juglans regia*); Getreide; Bäumen, z.B. Eichen *(Quercus),* bevorzugt die Fruchtbecher und/oder Früchte (*Valonea*), besonders bevorzugt von in Kleinasien und/oder auf dem südlichen Balkan wachsenden Eichenarten, und/oder Galläpfel (*Galla*), insbesondere von *Quercus infectoria*, *Quercus petraea* oder *Quercus robur,* Fichten (*Picea*)*,* dem Johannisbrotbaum (auch Bockshörndlbaum genannt) (*Ceratonia siliqua*)*,* dem Divi-Divi-Baum (*Caesalpinia coriaria*), dem Kaki-Baum (*Diospyros kaki*), Speierling (*Sorbus domestica*), Akazien (*Acacieae*), z.B. Gerberakazie (auch Katechu-Akazie genannt) (*Senegalia catechu*), Weide (*Salix*), Myrobalanenbaum, Mangrovenbaum (*Rhizophora*), Mangobaum (Mangifera indica), insbesondere Rinde, Fruchtfleisch und/oder Kern des Mangobaums, Hemlocktannen (auch Schierlingstanne genannt) (*Tsuga*), den Urunday-Baum (*Astronium balansae*), Birken (*Betula),* Elaeocarpus, insbesondere dessen Borke, Götterbaum (Ailanthus altissima), Kastanien (*Castanea*), insbesondere die Früchte und Blätter der Kastanie, der Betelnusspalme (*Areca catechu),* den Cashewbaumes (*Anacardium occidentale*) und/oder Quebracho (*Aspidosperma quebracho*blanco); Mimosen (*Mimosa*); Sumachgewächsen (*Rhus*), bevorzugt Gewürzsumach (*Rhus coriaria*), insbesondere dessen Früchte; Hopfen (*Humulus*); Obst, z.B. Äpfel (*Malus*), Birnen (Pyrus), Erdbeeren (*Fragaria*), Himbeeren (*Rubus idaeus*)*,* Brombeeren (*Rubus sect.* Rubus), Preiselbeeren (*Vaccinium vitis-idaea*), Bananen (Musa), Weintrauben (*Vitis vinifera*), Pfirsiche (*Prunus persica*), Quitten (*Cydonia oblonga*) und/oder Pflaumen (*Prunus domestica*)*;* Kaffeebohnen (z.B. *Coffea arabica oder Coffea canephora*); Teepflanzen (*Camellia sinensis*) wie Grüner Tee (z.B. japanische, chinesische und/oder indische Teesorten), insbesondere die Blätter von Grüner Tee Sencha, Schwarztee, insbesondere die Blätter von Darjeeling, Weißer Tee, inbesondere die Blätter von Weißer Tee China Oolong, und/oder Rooibos (*Aspalathus linearis*); Gewürznelken (Syzygium aromaticum), bevorzugt deren Knospen und/oder Blätter, der gemeinen Schafgarbe (*Achillea millefolium*); der Trauben-Silberkerze (*Actaea racemosa*); der Echten Bärentraube (*Arctostaphylos uva-ursi*); Estragon (*Artemisia dracunculus*)*,* Blutwurz (*Potentilla erecta*); und/oder Torf. Ferner kann bei den, insbesondere zerkleinerten und/oder getrockneten, Pflanzen oder Pflanzenbestandteilen zurückgegriffen werden auf Chebulische Myrobalane, Mangobaum (Mangifera indica), insbesondere die Rinde, Fruchtfleisch und Kern, laeocarpus (Elaecoarpus ganitrus), insbesondere die Borke, Langkapselige Jute (Corchorus olitorius), Rispen-Fuchsschwanz (Amaranthus eruentus), Augenbohne (Vigna unguiculata), Roter Mangold (Beta vulgaris var. cicla.), griechischer Bergtee (Sideritis raseri), Virginische Zaubernuss (Hamamelis virginiana), insbesondere die Rinde, Aleppo-Eiche, Färber-Eiche (Quercus infectoria), Korinthen (Vitis vinifera apyrena), Zichorien-Kaffee (Cichorium intybus), Carobpulver (Ceratonia siliqua), Grüner Hafer (Avena sativa L.), Ulmenrinde (Ulmus), Aroniabeerentrester (Aronia melanocarpa), Lindenrinde (Tilia), Holunderrinde (Sambucus nigra), Lapachorinde (Tabebuia impetiginosa), Chicorée (Cichorium intybus), Amla (Phyllanthus emblica), Chinesische Brombeere (Rubus suavissimus), Pomegranate (Punica granatum L.), Salbei (Salvia), Oregano (Origanum vulgare), Moltebeere (Rubus chamaemorus) und Dattelpalmen (Phoenix). Von der gemeinen Schafgarbe (*Achillea millefolium*), der Trauben-Silberkerze (*Actaea racemosa*), der Echten Bärentraube (*Arctostaphylos uva-ursi*), Estragon (*Artemisia dra*cunculus) und Blutwurz (*Potentilla erecta*) wird bevorzugt die gesamte Pflanze für die Extration verwendet. Bei der Betelnusspalme (*Areca catechu*) und dem Cashewbaum (*Anacardium occidentale*) greift man für die Extraktion bevorzugt auf deren Samen zurück.

Das erfindungsgemäße Verfahren umfasst gemäß der ersten Ausführungsvariante ferner (Schritt f)) das Abtrennen der, insbesondere zerkleinerten, Pflanzen oder Pflanzenbestandteile aus dem gemäß Schritt e) erhaltenen Produktgemisch, insbesondere im Anschluss an Schritt e), vorzugsweise mit Hilfe eines Siebs.

Zusätzlich umfasst das erfindungsgemäße Verfahren gemäß der ersten Ausführungsvariante außerdem (Schritt g)) das evaporative Konzentrieren des gemäß Schritt f) erhaltenen Produktgemisches, enthaltend Gallussäure im Anschluss an Schritt f) und vor Schritt h).

Auch umfasst das erfindungsgemäße Verfahren gemäß der ersten Ausführungsvariante ferner zusätzlich (Schritt h)) das Behandeln des Produktgemisches gemäß Schritt e) oder f) mit Ethanol und das Behandeln des Produktgemisches gemäß Schritt e) bzw. f) oder g) mit Wärme. Durch die Behandlung mit Ethanol und durch die Behandlung mit Wärme gelingt regelmäßig eine Deaktivierung des Enzyms Tannase. Dies erfolgt auch dadurch, dass man das Produktgemisch mit Ethanol in Schritt h) bei Temperaturen im Bereich von 40 bis 75 °C oder 48 bis 75 °C und bevorzugt 50 bis 70 °C behandelt. Zur weitergehenden Optimierung von Verfahrensschritt h) ist vorgesehen, dass in diesem Schritt zusätzlich Ethanol in einer Menge im Bereich von 40 bis 60 Gewichtsprozent, jeweils bezogen auf das Gesamtgewicht aus dem Ethanol und dem Wasser bzw. dem wässrigen System, zugegeben wird. Mit Schritt h) gelingt es regelmäßig sowohl, das Enzym zu deaktivieren, wie auch Phytoproteine und/oder Polysaccharide zu denaturieren und auszufällen.

In einer zweckmäßigen Ausgestaltung erfolgen die Schritte f) und g) aufeinander. An den Verfahrensschritt e) schließen sich die Verfahrensschritte f), g) h) an, wobei die Reihenfolge f)/g)/h) bevorzugt ist.

Das behandelte Produktgemisches gemäß Schritt h) wird gemäß der ersten Ausführungsvariante des erfindungsgemäßen Verfahrens filtriert, beispielsweise mit einem Filter mit einer Maschenweite im Bereich von 0,10 bis 1,0 µm. Auf diese Weise erhält man im Allgemeinen einen klaren Extrakt.

Das in Schritt h) zugegebene Ethanol wird anschließend wieder aus dem behandelten Produktgemisch gemäß Schritt h) oder dem filtrierten Produktgemisch gemäß Schritt i) entfernt (Schritt j).

Die extrahierende Behandlung gemäß Schritt e) wird bevorzugt bei Temperaturen im Bereich von 36 bis 38 °C durchgeführt.

Die der Erfindung zugrunde liegende Aufgabe wird des Weiteren gelöst durch ein Verfahren zur Herstellung eines Extrakts enthaltend Gallussäure (auch zweite Ausführungsvariante des erfindungsgemäßen Verfahrens genannt), umfassend die Schritte
1) Zurverfügungstellung von, insbesondere zerkleinerten und/oder, insbesondere und, getrockneten, Pflanzen oder Pflanzenbestandteilen, enthaltend Tannine, insbesondere Tanninsäure,
2) Zurverfügungstellung des Enzyms Tannase (IUB 3.1.1.20),
3) Zurverfügungstellung von Ethanol,
4) gegebenenfalls Zerkleinern bzw. weiteres Zerkleinern der Pflanzen oder Pflanzenbestandteile gemäß Schritt 1),
5) extrahierendes Behandeln der, insbesondere getrockneten und/oder zerkleinerten, Pflanzen oder Pflanzenbestandteile gemäß Schritt 1) und/oder 4), insbesondere oder, bei Temperaturen im Bereich von 50 bis 70 °C mit einem Gemisch enthaltend Ethanol und Wasser unter Erhalt eines Produktgemisches, enthaltend Polyphenole,
6) Abtrennen der, insbesondere zerkleinerten, Pflanzen oder Pflanzenbestandteile aus dem Produktgemisch gemäß Schritt 5) unter Erhalt eines Flüssigextrakts enthaltend die Polyphenole, insbesondere mit Hilfe eines Siebs,
7) Entfernen des Ethanols aus dem Flüssigextrakt gemäß Schritt 6) unter Erhalt eines wässrigen Extrakts enthaltend die Polyphenole,
8) Behandeln des wässrigen Extrakts gemäß Schritt 7) mit dem Enzym Tannase (IUB 3.1.1.20) bei Temperaturen im Bereich von 35 bis 40 °C unter Erhalt eines wässrigen Systems, enthaltend Gallussäure,
9) evaporatives Konzentrieren des wässrigen Systems, enthaltend Gallussäure, gemäß Schritt 8) unter Erhalt eines wässrigen Konzentrats,
   wobei in Schritt 5) Ethanol in einer Menge im Bereich von 60 bis 90 Gewichtsprozent, bezogen auf das Gesamtgewicht aus Ethanol und dem Wasser, zugegeben wird,
   wobei die, insbesondere gemahlenen, vorzugsweise zerkleinerten, Pflanzen oder Pflanzenbestandteile in Schritt 5) immobilisiert vorliegen und
   wobei das extrahierende Behandeln gemäß Schritt 5) ein Perkolationsverfahren umfasst.

Die der Erfindung zugrunde liegende Aufgabe wird mit dem Verfahren gemäß der zweiten Ausführungsvariante besonders gut auch dadurch gelöst, dass man das extrahierende Behandeln gemäß Schritt 5) bei Temperaturen im Bereich von 55 bis 65 °C durchführt.

In einer bevorzugten Ausgestaltung zeichnet sich die zweiter Ausführungsvariante des erfindungsgemäßen Verfahrens auch dadurch aus, dass in Schritt 5) Ethanol in einer Menge im Bereich von 70 bis 85 Gewichtsprozent, bezogen auf das Gesamtgewicht aus Ethanol und dem Wasser, zugegeben wird.

Hierbei wird in der zweiten Ausführungsvariante des erfindungsgemäßen Verfahrens das Behandeln des wässrigen Extrakts gemäß Schritt 8) mit dem Enzym Tannase bevorzugt bei Temperaturen im Bereich von 36 bis 38 °C durchgeführt.

Überraschend hat sich gezeigt, dass eine besonders effiziente Verfahrensführung auch dadurch gelingt, dass die, insbesondere gemahlenen, vorzugsweise zerkleinerten, Pflanzen oder Pflanzenbestandteile in Schritt e) gemäß der ersten Ausführungsvariante bzw. Schritt

5) gemäß der zweiten Ausführungsvariante immobilisiert, insbesondere in einem Siebträger immobilisiert, vorliegen.

Das extrahierende Behandeln gemäß Schritt e) in der ersten Ausführungsvariante bzw. Schritt 5) in der zweiten Ausführungsvariante umfasst ein Perkolationsverfahren oder stellt ein solches dar.

Besonders hohe Ausbeuten bzw. Konzentrationen an Gallussäure lassen sich mit den erfindungsgemäßen Verfahren auch dadurch erreichen, dass man die Menge an Tannase in Schritt e) gemäß der ersten Ausführungsvariante bzw. Schritt 8) gemäß der zweiten Ausführungsvariante im Bereich von 0,1 U/g bis 100,0 U/g, bevorzugt im Bereich von 0,2 U/g bis 50,0 U/g, bezogen auf die Menge an Pflanzen oder Pflanzenbestandteilen, einstellt.

Der nach Schritt i) oder j) erhaltene wässrige Extrakt, enthaltend Gallussäure, gemäß der ersten Ausführungsvariante bzw. das wässrige System, enthaltend Gallussäure, gemäß Schritt 8) gemäß der zweiten Ausführungsvariante wird evaporativ aufzukonzentriert unter Erhalt eines wässrigen Konzentrats. Hierbei handelt sich bevorzugt um einen sogenannten Spissumextrakt. Dieses wässrige Konzentrat bzw. dieser Spissumextrakt verfügt dabei insbesondere über einen Brix-Wert im Bereich von 1° bis 100 ° Brix, bevorzugt im Bereich von 10° bis 70° Brix und besonders bevorzugt im Bereich von 20° bis 50° Brix. Ganz besonders bevorzugt werden bei dem Schritt des Aufkonzentrierens Konzentrate mit Brix-Werten im Bereich von 20° bis 40° Brix, insbesondere im Bereich von 25° bis 35° Brix, angestrebt bzw. erhalten. Die Bestimmung von Grad Brix ist dem Fachmann geläufig. Eine Flüssigkeit weist einen Brix-Wert von 1° auf, wenn sie dieselbe Dichte hat wie eine Lösung von 1 g Saccharose in 100 g Saccharose/Wasser-Lösung, und einen Brix-Wert von 10°, wenn ihre Dichte die einer Lösung von 10 g Saccharose in 100 g Saccharose-Wasser-Lösung beträgt. Somit bedeutet die Angabe in Grad Brix, dass die Dichte der gemessenen Flüssigkeit der Dichte einer Lösung von Saccharose in Wasser entspricht, die so viele Gramm Saccharose pro 100 g Lösung enthält, wie die Gradangabe nennt.

Die der Erfindung zugrunde liegende Aufgabe wird des Weiteren gelöst durch ein wässriges Konzentrat gemäß Anspruch 10 enthaltend Gallussäure, erhalten nach der ersten Ausführungsvariante, wobei die für dieses wässrige Konzentrat verwendete Menge an Trockenmasse im Bereich von 5 bis 80 Gewichtsprozent, bevorzugt im Bereich von 10 bis 50 Gewichtsprozent und besonders bevorzugt im Bereich von 15 bis 40 Gewichtsprozent, jeweils bezogen auf das Gesamtgewicht des wässrigen Konzentrats, liegt. Diese wässrigen Konzentrate eignen sich insbesondere für die Herstellung von Lebensmitteln und Nahrungsergänzungsmitteln, insbesondere von solchen in Form von Getränken.

Unter Trockenmasse im Sinne der Erfindung soll diejenige Trockenmasse verstanden werden, die man durch Entfernen sämtlicher wässriger und nicht-wässriger Lösungsmittel aus den nach den erfindungsgemäßen Verfahren zugänglichen Extrakten erhält.

Die wässrigen Konzentrate gemäß Anspruch 10 werden nach dem erfindungsgemäßen Verfahren gemäß der ersten Ausführungsvariante oder nach dem erfindungsgemäßen Verfahren gemäß der zweiten Ausführungsvariante erhalten, wobei diejenigen wässrigen Konzentrate besonders bevorzugt sind, die nach dem erfindungsgemäßen Verfahren gemäß der ersten Ausführungsvariante zugänglich sind.

Die erfindungsgemäßen wässrigen Konzentrate gemäß Anspruch 10, , insbesondere erhalten nach der ersten Ausführungsvariante, verfügen über einen pH-Wert im Bereich von 1,5 bis 5,5, bevorzugt im Bereich von 1,75 bis 4,5 und besonders bevorzugt im Bereich von 2,0 bis 4,0, bzw. sind auf einen pH-Wert im Bereich von 1,5 bis 5,5, bevorzugt im Bereich von 1,75 bis 4,5 und besonders bevorzugt im Bereich von 2,0 bis 4,0, eingestellt.

In den erfindungsgemäßen wässrigen Konzentraten gemäß Anspruch 10, insbesondere erhalten nach der ersten Ausführungsvariante, beträgt die Menge an Gallussäure mindestens 10 g/kg Trockenmasse, bevorzugt mindestens 50 g/kg Trockenmasse und besonders bevorzugt mindestens 75 g/kg Trockenmasse, und/oder bei denen Gallussäure in einer Menge im Bereich von 10 bis 250 g/kg Trockenmasse, bevorzugt im Bereich von 50 bis 200 g/kg Trockenmasse und besonders bevorzugt im Bereich von 75 bis 175 g/kg Trockenmasse, vorliegt, jeweils bezogen auf das Gesamtgewicht des wässrigen Konzentrats.

Erfindungsgemäße wässrige Konzentrate gemäß Anspruch 10, insbesondere erhalten nach der ersten Ausführungsvariante, verfügen dabei über eine relative Dichte [20/20] im Bereich von 1,001 bis 1,4000, bevorzugt im Bereich von 1,010 bis 1,2500. Die Relative Dichte [20/20] ist der Quotient aus der Dichte der untersuchten Probe (gemessen bei 20°C) und der Dichte von Wasser bei 20°C. Die Messung erfolgt hierbei insbesondere mittels Biegeschwinger nach § 35 LMBG 36.0 3a (deutsches Lebensmittel- und Bedarfsgegenständegesetz in der zum Anmeldetag geltenden Fassung) (Bestimmung der relativen Dichte d 20/20 von Würze und Bier (Biegeschwinger-Verfahren). Besonders geeignete wässrige Konzentrate zeichnen sich durch einen Gesamtphenolgehalt im Bereich von 5 bis 350 g GAE/kg, bevorzugt im Bereich von 50 bis 300 g GAE/kg und besonders bevorzugt im Bereich von 75 bis 250 g GAE/kg sowie insbesondere im Bereich von 125 bis 200 g GAE/kg aus, jeweils bezogen auf die Trockenmasse des Extrakts.

Der Gesamtphenolgehalt kann hierbei nach der Methode gemäß DIN ISO 14502-1:2007-11 (Bestimmung von charakteristischen Substanzen von grünem und schwarzem Tee - Teil 1: Gesamt-Polyphenolgehalt in Tee - Colorimetrisches Verfahren mit Folin-Ciocalteu-Reagenz) ermittelt werden.

Mit der vorliegenden Erfindung geht die überraschende Erkenntnis einher, dass sich bei Einsatz der erfindungsgemäßen Extraktionsverfahren gemäß der aus ersten Ausführungsvariante wie auch gemäß der zweiten Ausführungsvariante, insbesondere jedoch gemäß der ersten Ausführungsvariante, Gallussäure in hohen Ausbeuten effektiv und kostengünstig aus vielfältigen pflanzlichen Materialien bzw. Bestandteilen hiervon gewinnen lässt, wodurch wässrige Konzentrate mit einer sehr hohen Gallussäure-Konzentration zugänglich sind. Hierbei wurde überraschend festgestellt, dass die erfindungsgemäßen wässrigen Konzentrate ausgestattet sind mit einer Trolox-äquivalenten antioxidativen Kapazität im Bereich von 500 bis 6000 µmol/g, bevorzugt im Bereich von 1000 bis 5000 µmol/g und besonders bevorzugt von 2000 bis 4000 µmol/g, jeweils bezogen auf die Trockenmasse des Extrakts. Die Trolox-äquivalente antioxidative Kapazität kann zweckmäßigerweise mit dem DPPH (2,2-diphenyl-1-picrylhydrazyl)-Assay bestimmt werden. Dieses Bestimmungsverfahren ist dem Fachmann geläufig und wird beispielsweise beschrieben von O. Sharma und T. N. Bhat in "DPPH antioxidant assay revisited", Food Chemistry 113(4):1202-1205, April 2009, sowie von A. Karioti et al. in "Composition and antioxidant activity of the essential oils of Xylopia aethiopica (Dun) A. Rich. (Annonaceae) leaves, stem bark, root bark, and fresh and dried fruits, growing in Ghana", J. Agric. Food Chem. 29;52(26):8094-8, December 2004. Beispielsweise können bei diesem Verfahren 10 mg des stabilen Radikals DPPH in 100 ml Methanol gelöst und nach Mischen mit dem jeweiligen Probenextrakt bzw. dem Standard (Trolox) für 30 min bei Raumtemperatur inkubiert sowie anschließend die Extinktion photometrisch bei 517 nm bestimmt werden. Das antioxidative Potential der Proben kann dann aus einer linearen Beziehung zwischen Absorption und den Standardkonzentrationen bestimmt werden. Die Ergebnisse lassen sich als Troloxäquivalente bzw. als Mikromol Trolox/g Probe berechnen sowie in Bezug zur Trockenmasse setzen. Mit dem beschriebenen Verfahren wird eine Extinktions-Differenz ermittelt, aus der durch Vergleich mit der durch Trolox in verschiedenen Konzentrationen verursachten Minderung der Extinktion die antioxidative Kapazität der untersuchten Substanz ermittelt werden kann.

Die erfindungsgemäßen wässrigen Konzentrate können unmittelbar für die Herstellung von Lebensmitteln, insbesondere Getränken, und Nahrungsergänzungsmittel verwendet werden.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Verfahren zur Herstellung eines Extrakts enthaltend Gallussäure, umfassend die Schritte
a) Zurverfügungstellung von, insbesondere getrockneten und/oder, insbesondere und, zerkleinerten, Pflanzen oder Pflanzenbestandteilen, enthaltend Tannine, insbesondere Tanninsäure,
b) Zurverfügungstellung des Enzyms Tannase,
c) Zurverfügungstellung von Ethanol,
d) gegebenenfalls Zerkleinern bzw. weiteres Zerkleinern der Pflanzen oder Pflanzenbestandteile gemäß Schritt a),
e) extrahierendes Behandeln der gemäß Schritt a) und/oder d) erhaltenen, insbesondere zerkleinerten, Pflanzen oder Pflanzenbestandteile mit Wasser in Gegenwart des Enzyms Tannase bei Temperaturen im Bereich von 35 bis 40 °C unter Erhalt eines Produktgemisches enthaltend Gallussäure,
f) das Abtrennen der, insbesondere zerkleinerten, Pflanzen oder Pflanzenbestandteile aus dem gemäß Schritt e) erhaltenen Produktgemisches, insbesondere im Anschluss an Schritt e) und
g) das evaporative Konzentrieren des gemäß Schritt f) erhaltenen Produktgemisches, enthaltend Gallussäure im Anschluss an Schritt f) und vor Schritt h) und
h) Behandeln des Produktgemisches gemäß Schritt e) oder f) mit Ethanol und Behandeln des Produktgemisches gemäß Schritt e) oder f) oder g) mit Wärme bei Temperaturen im Bereich von 40 bis 75 °C, wobei Schritt h) bewirkt, das Enzym Tannase zu deaktivieren, wie auch Proteine und/oder Polysaccharide zu denaturieren und auszufällen, und
i) Filtrieren des behandelten Produktgemisches gemäß Schritt h) und,
j) Entfernen des in Schritt h) zugegebenen Ethanols aus dem behandelten Produktgemisch gemäß Schritt h) oder dem filtrierten Produktgemisch gemäß Schritt i),
k) evaporatives Konzentrieren des nach Schritt j) erhaltenen wässrigen Extrakts, enthaltend Gallussäure unter Erhalt eines wässrigen Konzentrats,
wobei in Schritt h) Ethanol in einer Menge im Bereich von 40 bis 60 Gewichtsprozent, bezogen auf das Gesamtgewicht aus dem Ethanol und dem Wasser bzw. dem wässrigen System, zugegeben wird, wobei die, insbesondere gemahlenen, vorzugsweise zerkleinerten, Pflanzen oder Pflanzenbestandteile in Schritt e) immobilisiert vorliegen und
wobei das extrahierende Behandeln gemäß Schritt e) ein Perkolationsverfahren umfasst.

2. Verfahren nach Anspruch 1, **dadurch gekennzeichnet, dass**
das extrahierende Behandeln gemäß Schritt e) bei Temperaturen im Bereich von 36 bis 38 °C durchgeführt wird.

3. Verfahren zur Herstellung eines Extrakts enthaltend Gallussäure, umfassend die Schritte
1) Zurverfügungstellung von, insbesondere zerkleinerten und/oder, insbesondere und, getrockneten, Pflanzen oder Pflanzenbestandteilen, enthaltend Tannine, insbesondere Tanninsäure,
2) Zurverfügungstellung des Enzyms Tannase,
3) Zurverfügungstellung von Ethanol,
4) gegebenenfalls Zerkleinern bzw. weiteres Zerkleinern der Pflanzen oder Pflanzenbestandteile gemäß Schritt 1),
5) extrahierendes Behandeln der, insbesondere zerkleinerten, Pflanzen oder Pflanzenbestandteile gemäß Schritt 1) und/oder 4) bei Temperaturen im Bereich von 50 bis 70 °C mit einem Gemisch enthaltend Ethanol und Wasser unter Erhalt eines Produktgemisches, enthaltend Polyphenole,
6) Abtrennen der, insbesondere zerkleinerten, Pflanzen oder Pflanzenbestandteile aus dem Produktgemisch gemäß Schritt 5) unter Erhalt eines Flüssigextrakts enthaltend die Polyphenole,
7) Entfernen des Ethanols aus dem Flüssigextrakt gemäß Schritt 6) unter Erhalt eines wässrigen Extrakts enthaltend die Polyphenole,
8) Behandeln des wässrigen Extrakts gemäß Schritt 7) mit dem Enzym Tannase bei Temperaturen im Bereich von 35 bis 40 °C unter Erhalt eines wässrigen Systems, enthaltend Gallussäure,
9) evaporatives Konzentrieren des wässrigen Systems, enthaltend Gallussäure, gemäß Schritt 8) unter Erhalt eines wässrigen Konzentrats,
wobei in Schritt 5) Ethanol in einer Menge im Bereich von 60 bis 90 Gewichtsprozent, bezogen auf das Gesamtgewicht aus Ethanol und dem Wasser, zugegeben wird,
wobei die, insbesondere gemahlenen, vorzugsweise zerkleinerten, Pflanzen oder Pflanzenbestandteile in Schritt 5) immobilisiert vorliegen und
wobei das extrahierende Behandeln gemäß Schritt 5) ein Perkolationsverfahren umfasst.

4. Verfahren nach einem der vorangehenden Ansprüche, **dadurch gekennzeichnet, dass**
die, insbesondere gemahlenen, vorzugsweise zerkleinerten, Pflanzen oder Pflanzenbestandteile in Schritt e) bzw. Schritt 5) in einem Siebträger immobilisiert vorliegen.

5. Verfahren nach einem der vorangehenden Ansprüche, **dadurch gekennzeichnet, dass**
die Menge an Tannase in Schritt e) bzw. Schritt 8) im Bereich von 0,1 U/g bis 100,0 U/g, bevorzugt im Bereich von 0,2 U/g bis 50,0 U/g, bezogen auf die Menge an Pflanzen oder Pflanzenbestandteilen, liegt.

6. Verfahren nach einem der vorangehenden Ansprüche, **dadurch gekennzeichnet, dass** das wässrige Konzentrat, erhalten durch evaporatives Konzentrieren des nach Schritt k), erhaltenen wässrigen Extrakts, enthaltend Gallussäure, bzw. des wässrigen Systems, enthaltend Gallussäure, gemäß Schritt 9) ein Spissumextrakt darstellt.

7. Verfahren nach Anspruch 6, **dadurch gekennzeichnet, dass**
das wässrige Konzentrat einen Brix-Wert im Bereich von 1° bis 100 ° Brix, bevorzugt im Bereich von 10° bis 70° Brix und besonders bevorzugt im Bereich von 20° bis 50° Brix, aufweist.

8. Verfahren nach einem der Ansprüche 3 bis 7, **dadurch gekennzeichnet, dass** das extrahierende Behandeln gemäß Schritt 5) bei Temperaturen im Bereich von 55 bis 65 °C durchgeführt wird.

9. Verfahren nach einem der Ansprüche 3 bis 8, **dadurch gekennzeichnet, dass** in Schritt 5) Ethanol in einer Menge im Bereich von 70 bis 85 Gewichtsprozent, bezogen auf das Gesamtgewicht aus Ethanol und dem Wasser, zugegeben wird.

10. Wässriges Konzentrat, enthaltend Gallussäure, erhalten oder erhältlich gemäß einem Verfahren nach einem der vorangehenden Ansprüche, **gekennzeichnet durch** eine Trockenmasse im Bereich von 5 bis 80 Gewichtsprozent, bevorzugt im Bereich von 10 bis 50 Gewichtsprozent und besonders bevorzugt im Bereich von 15 bis 40 Gewichtsprozent, jeweils bezogen auf das Gesamtgewicht des wässrigen Konzentrats, durch einen pH-Wert im Bereich von 1,5 bis 5,5, bevorzugt im Bereich von 1,75 bis 4,5 und besonders bevorzugt im Bereich von 2,0 bis 4,0, durch eine Menge an Gallussäure von mindestens 10 g/kg Trockenmasse, bevorzugt mindestens 50 g/kg Trockenmasse und besonders bevorzugt mindestens 75 g/kg Trockenmasse, und/oder eine Menge an Gallussäure im Bereich von 10 bis 250 g/kg Trockenmasse, bevorzugt im Bereich von 50 bis 200 g/kg Trockenmasse und besonders bevorzugt im Bereich von 75 bis 175 g/kg Trockenmasse, und durch eine relative Dichte [20/20] im Bereich von 1,001 bis 1,400, bevorzugt im Bereich von 1,0100 bis 1,2500, ermittelt mittels Biegeschwinger-Verfahren nach dem in den Beschreibung genannten Verfahren, wobei das wässrige Konzentrat eine Trolox-äquivalente antioxidative Kapazität im Bereich von 500 bis 6000 µmol/g, bezogen auf die Trockenmasse des Extrakts, aufweist, bestimmt mit dem DPPH (2,2-diphenyl-1-picrylhydrazyl)-Assay nach dem in der Beschreibung genannten Bestimmungsverfahren.

11. Verwendung des wässrigen Konzentrats gemäß Anspruch 10 in einem Lebensmittel, insbesondere Getränk, oder Nahrungsergänzungsmittel.

## Claims

1. A method for preparing an extract containing gallic acid, comprising the steps
a) providing, in particular dried and/or, in particular and, comminuted, plants or plant constituents containing tannins, in particular tannic acid,
b) providing the enzyme tannase,
c) providing ethanol,
d) optionally comminuting or further comminuting the plants or plant constituents according to step a),
e) performing an extraction treatment on the, in particular comminuted, plants or plant constituents obtained according to step a) and/or d) by way of water in the presence of the enzyme tannase at temperatures in the range from 35 to 40°C to obtain a product mixture containing gallic acid,
f) separating the, in particular comminuted, plants or plant constituents from the product mixture obtained according to step e), in particular after step e), and
g) evaporative concentration of the product mixture containing gallic acid obtained according to step f) after step f) and before step h), and
h) treating the product mixture according to step e) or f) with ethanol, and treating the product mixture according to step e) or f) or g) with heat at temperatures in the range from 40 to 75°C, wherein step h) has the effect of deactivating the enzyme tannase and also of denaturing and precipitating proteins and/or polysaccharides, and
i) filtering the treated product mixture according to step h), and
j) removing the ethanol added in step h) from the treated product mixture according to step h) or the filtered product mixture according to step i),
k) evaporative concentration of the aqueous extract containing gallic acid obtained after step j) to obtain an aqueous concentrate,
wherein ethanol is added in step h) in an amount in the range from 40 to 60 percent by weight, based on the total weight of the ethanol and the water or the aqueous system, wherein the, in particular ground, preferably comminuted, plants or plant constituents are present in immobilized form in step e), and
wherein the extraction treatment according to step e) comprises a percolation process.

2. The method according to claim 1, **characterized in that**
the extraction treatment according to step e) is carried out at temperatures in the range from 36 to 38°C.

3. A method for preparing an extract containing gallic acid, comprising the steps
1) providing, in particular comminuted and/or, in particular and, dried, plants or plant constituents containing tannins, in particular tannic acid,
2) providing the enzyme tannase,
3) providing ethanol,
4) optionally comminuting or further comminuting the plants or plant constituents according to step 1),
5) performing an extraction treatment on the, in particular comminuted, plants or plant constituents according to step 1) and/or 4) at temperatures in the range from 50 to 70°C with a mixture containing ethanol and water to obtain a product mixture containing polyphenols,
6) separating the, in particular comminuted, plants or plant constituents from the product mixture according to step 5) to obtain a liquid extract containing the polyphenols,
7) removing the ethanol from the liquid extract according to step 6) to obtain an aqueous extract containing the polyphenols,
8) treating the aqueous extract according to step 7) with the enzyme tannase at temperatures in the range from 35 to 40°C to obtain an aqueous system containing gallic acid,
9) evaporative concentration of the aqueous system containing gallic acid according to step 8) to obtain an aqueous concentrate,
wherein ethanol is added in step 5) in an amount in the range from 60 to 90 percent by weight, based on the total weight of ethanol and the water,
wherein the, in particular ground, preferably comminuted, plants or plant constituents are present in immobilized form in step 5), and
wherein the extraction treatment according to step 5) comprises a percolation process.

4. The method according to any one of the preceding claims, **characterized in that** the, in particular ground, preferably comminuted, plants or plant constituents are immobilized in step e) or step 5) in a sieve support.

5. The method according to any one of the preceding claims, **characterized in that** the amount of tannase in step e) or step 8) is in the range from 0.1 U/g to 100.0 U/g, preferably in the range from 0.2 U/g to 50.0 U/g, based on the amount of plants or plant constituents.

6. The method according to any one of the preceding claims, **characterized in that** the aqueous concentrate obtained by evaporative concentration of the aqueous extract containing gallic acid obtained after step k) or the aqueous system containing gallic acid according to step 9) is a spissum extract.

7. The method according to claim 6, **characterized in that**
the aqueous concentrate has a Brix value in the range from 1° to 100° Brix, preferably in the range from 10° to 70° Brix and particularly preferably in the range from 20° to 50° Brix.

8. The method according to any one of claims 3 to 7, **characterized in that** the extraction treatment according to step 5) is carried out at temperatures in the range from 55 to 65°C.

9. The method according to any one of claims 3 to 8, **characterized in that** ethanol is added in step 5) in an amount in the range from 70 to 85 percent by weight, based on the total weight of ethanol and the water.

10. An aqueous concentrate containing gallic acid, obtained or obtainable according to a method according to any one of the preceding claims, **characterized by** a dry weight in the range from 5 to 80 percent by weight, preferably in the range from 10 to 50 percent by weight and particularly preferably in the range from 15 to 40 percent by weight, in each case based on the total weight of the aqueous concentrate, by a pH value in the range from 1.5 to 5.5, preferably in the range from 1.75 to 4.5 and particularly preferably in the range from 2.0 to 4.0, by an amount of gallic acid of at least 10 g/kg dry weight, preferably at least 50 g/kg dry weight and particularly preferably at least 75 g/kg dry weight, and/or an amount of gallic acid in the range from 10 to 250 g/kg dry weight, preferably in the range from 50 to 200 g/kg dry weight and particularly preferably in the range from 75 to 175 g/kg dry weight, and by a relative density [20/20] in the range from 1.001 to 1.400, preferably in the range from 1.0100 to 1.2500, determined by way of oscillating u-shape method according to the method mentioned in the description, wherein the aqueous concentrate has a Trolox-equivalent antioxidant capacity in the range from 500 to 6000 µmol/g, based on the dry weight of the extract, determined with the DPPH (2,2-diphenyl-1-picrylhydrazyl) assay according to the determination method mentioned in the description.

11. Use of the aqueous concentrate according to claim 10 in a food, in particular drink, or food supplement.

## Revendications

1. Procedé de fabrication d'un extrait contenant de l'acide gallique, comprenant les étapes suivantes :
a) mise à disposition de, en particulier séchées et/ou, en particulier et, broyées, plantes ou constituants végétaux, contenant des tanins, en particulier de l'acide tannique,
b) mise à disposition de l'enzyme tannase,
c) mise à disposition d'éthanol,
d) le cas échéant, broyage ou broyage supplémentaire des plantes ou constituants végétaux selon l'étape a),
e) traitement extractif des plantes ou constituants végétaux, en particulier broyés, obtenus selon l'étape a) et/ou d), avec de l'eau en présence de l'enzyme tannase à des températures comprises entre 35 et 40 °C, obtenant un mélange de produits contenant de l'acide gallique,
f) séparation des plantes ou constituants végétaux, en particulier broyés, du mélange de produits obtenu selon l'étape e), en particulier à la suite de l'étape e), et
g) concentration évaporative du mélange de produits obtenu selon l'étape f), contenant de l'acide gallique, à la suite de l'étape f) et avant l'étape h), et
h) traitement du mélange de produits selon l'étape e) ou f) avec de l'éthanol et traitement du mélange de produits selon l'étape e) ou f) ou g) avec de la chaleur à des températures comprises entre 40 et 75 °C, l'étape h) permettant de désactiver l'enzyme tannase, ainsi que de dénaturer et de précipiter des protéines et/ou des polysaccharides, et
i) filtration du mélange de produits traité selon l'étape h), et,
j) élimination de l'éthanol ajouté à l'étape h) du mélange de produits traité selon l'étape h) ou du mélange de produits filtré selon l'étape i),
k) concentration évaporative de l'extrait aqueux obtenu après l'étape j), contenant de l'acide gallique, obtenant un concentré aqueux, l'éthanol étant ajouté à l'étape h) en une quantité comprise entre 40 et 60 pour cent en poids, par rapport au poids total de l'éthanol et de l'eau ou du système aqueux, les plantes ou constituants végétaux moulus, de préférence broyés, à l'étape e) étant présents sous forme immobilisée et le traitement extractif selon l'étape e) comprenant un procedé de percolation.

2. Procédé selon la revendication 1, **caractérisé en ce que** le traitement extractif selon l'étape e) est effectué à des températures comprises entre 36 et 38 °C.

3. Procedé de fabrication d'un extrait contenant de l'acide gallique, comprenant les étapes suivantes :
1) mise à disposition de, en particulier broyées et/ou, en particulier et, séchées, plantes ou constituants végétaux, contenant des tanins, en particulier de l'acide tannique,
2) mise à disposition de l'enzyme tannase,
3) mise à disposition d'éthanol,
4) le cas échéant, broyage ou broyage supplémentaire des plantes ou constituants végétaux selon l'étape 1),
5) traitement extractif des plantes ou constituants végétaux, en particulier broyés, selon l'étape 1) et/ou 4) à des températures comprises entre 50 et 70 °C avec un mélange contenant de l'éthanol et de l'eau, obtenant un mélange de produits contenant des polyphénols,
6) séparation des plantes ou constituants végétaux, en particulier broyés, du mélange de produits selon l'étape 5), obtenant un extrait liquide contenant les polyphénols,
7) élimination de l'éthanol de l'extrait liquide selon l'étape 6), obtenant un extrait aqueux contenant les polyphénols,
8) traitement de l'extrait aqueux selon l'étape 7) avec l'enzyme tannase à des températures comprises entre 35 et 40 °C, obtenant un système aqueux contenant de l'acide gallique,
9) concentration évaporative du système aqueux contenant de l'acide gallique selon l'étape 8), obtenant un concentré aqueux, l'éthanol étant ajouté à l'étape 5) en une quantité comprise entre 60 et 90 pour cent en poids, par rapport au poids total de l'éthanol et de l'eau, les plantes ou constituants végétaux moulus, de préférence broyés, à l'étape 5) étant présents sous forme immobilisée et le traitement extractif selon l'étape 5) comprenant un procédé de percolation.

4. Procedé selon l'une des revendications précédentes, **caractérisé en ce que** les plantes ou constituants végétaux moulus, de préférence broyés, à l'étape e) ou à l'étape 5) sont présents sous forme immobilisée dans un porte-tamis.

5. Procédé selon l'une des revendications précédentes, **caractérisé en ce que** la quantité de tannase à l'étape e) ou à l'étape 8) est comprise entre 0,1 U/g et 100,0 U/g, de préférence entre 0,2 U/g et 50,0 U/g, par rapport à la quantité de plantes ou de constituants végétaux.

6. Procédé selon l'une des revendications précédentes, **caractérisé en ce que** le concentré aqueux, obtenu par concentration évaporative de l'extrait aqueux obtenu après l'étape k), contenant de l'acide gallique, ou du système aqueux contenant de l'acide gallique selon l'étape 9), constitue un extrait spissum.

7. Procédé selon la revendication 6, **caractérisé en ce que** le concentré aqueux présente une valeur Brix comprise entre 1° et 100° Brix, de préférence entre 10° et 70° Brix et particulièrement de préférence entre 20° et 50° Brix.

8. Procédé selon l'une des revendications 3 à 7, **caractérisé en ce que** le traitement extractif selon l'étape 5) est effectué à des températures comprises entre 55 et 65 °C.

9. Procédé selon l'une des revendications 3 à 8, **caractérisé en ce que** à l'étape 5), l'éthanol est ajouté en une quantité comprise entre 70 et 85 pour cent en poids, par rapport au poids total de l'éthanol et de l'eau.

10. Concentré aqueux, contenant de l'acide gallique, obtenu ou obtenable selon un procédé selon l'une des revendications précédentes, **caractérisé par** une matière sèche comprise entre 5 et 80 pour cent en poids, de préférence entre 10 et 50 pour cent en poids et particulièrement de préférence entre 15 et 40 pour cent en poids, chaque fois par rapport au poids total du concentré aqueux, par une valeur de pH comprise entre 1,5 et 5,5, de préférence entre 1,75 et 4,5 et particulièrement de préférence entre 2,0 et 4,0, par une quantité d'acide gallique d'au moins 10 g/kg de matière sèche, de préférence au moins 50 g/kg de matière sèche et particulièrement de préférence au moins 75 g/kg de matière sèche, et/ou une quantité d'acide gallique comprise entre 10 et 250 g/kg de matière sèche, de préférence entre 50 et 200 g/kg de matière sèche et particulièrement de préférence entre 75 et 175 g/kg de matière sèche, et par une densité relative [20/20] comprise entre 1,001 et 1,400, de préférence entre 1,0100 et 1,2500, déterminée selon la méthode à oscillateur à tube vibrant décrite dans la description, le concentré aqueux présentant une capacité antioxydante équivalente Trolox comprise entre 500 et 6000 µmol/g, par rapport à la matière sèche de l'extrait, déterminée avec le test DPPH (2,2-diphényl-1-picrylhydrazyl) selon la méthode de détermination décrite dans la description.

11. Utilisation du concentré aqueux selon la revendication 10 dans un aliment, en particulier une boisson, ou un complément alimentaire.
